# NEUE EUROPÄISCHE PATENTSCHRIFT

(11) **EP 0 346 582 B2**
(45) Veröffentlichungstag und Bekanntmachung des Hinweises auf die Entscheidung über den Einspruch: **30.08.1995**
(45) Hinweis auf die Patenterteilung: 31.03.1993
(21) Anmeldenummer: 89106461.0
(22) Anmeldetag: 12.04.1989
(51) Int. Cl.: A61K 7/06, A61K 7/08, A61K 7/00

(54) **Haarpflegeemulsion**
Haircare emulsion
Emulsion pour le soin des cheveux

(30) Priorität: 15.06.1988 DE 3820330
(43) Veröffentlichungstag der Anmeldung: 20.12.1989
(73) Patentinhaber: Wella Aktiengesellschaft, 64295 Darmstadt (DE)
(72) Erfinder: Gross, Paul, D-6100 Darmstadt (DE); Keller, Walter, D-6105 Ober-Ramstadt (DE); Bräunig, Helga, D-6112 Gross-Zimmern (DE)

(56) Entgegenhaltungen:
- EP-A- 0 218 441
- EP-A- 0 276 151
- GB-A- 2 114 988
- US-A- 3 415 607
- PATENT ABSTRACTS OF JAPAN, Band 13, Nr. 72 (C-570)[3420], 17. Februar 1989
- SOVIET INVENTIONS ILLUSTRATED, Woche 8542, 22. November 1985, Nr. 85-261673/42, Derwent Publications Ltd, London, GB
- CHEMICAL ABSTRACTS, Band 76, 1972, Seite 306, Zusammenfassung Nr. 131382t, Columbus, Ohio, US; R. WOODFORD et al.: "Effect of ethanol and propylene glycol, and a mixture of potassium sorbate with either, on Pseudomonas aeruginose contamination of an oil-in-water cream"
- H. JANISTYN: "Handbuch der Kosmetika und Riechstoffe", Auflage 2, Band 3: "Die Körperpflegemittel", 1973, Seiten 413-419, Dr. Alfred Hüthig Verlag, Heidelberg, DE
- SOAP, PERFUMERY AND COSMETICS, Band 46, Nr. 7, Juli 1973, Seiten 409-418; J.G. DAVIS: "The microbiological stability of cosmetic and toilet preparations"
- Produktinformationsblatt W1 2557/5 der Hoechst AG, 7/87 und Bestätigungsschreiben der Hoechst AG v. 17.12.93 zur allgemeinen Zugänglichkeit des Informationsblattes
- Pharmazie Modell-Rezepturen, herausgegeben von Henkel-Dehydag, Ausgabe 1983, S. 63, Rezeptur-Nr. Ph-A 22.07
- K. Schrader, Grundlagen und Rezepturen der Kosmetika, 1979, A. Hüthig Verlag, S. 86, 101
- K. Wallhäusser, Praxis der Sterilisation-Desinfektion-Konservierung, 4. Auflage 1988, G. Thieme Verlag, S. 428, 429, 431, 433
- K. Wallhäusser, Sterilisation Desinfektion Konservierung, 2. Auflage 1978, 392-394
- Duden Fremdwörtebuch, 2. Auflage 1971, S. 589-591
- Lexikon der Medizin (Herausg. D. Tutsch), 2. Auflage 1981, S. 469, Verlag Urban & Schwarzenberg
- Webster's II New Riverside University Dictionary 1984, S. 1158, 1351
- K.H. Wallhäusser, Sterilisation, Desinfektion, Konservierung, 2. Auflage 1978, S. 413, 421

## Beschreibung

Gegenstand der Erfindung ist eine aniontensidhaltige Haarpflegeemulsion, die nach einer ausreichenden Einwirkungszeit wieder aus dem Haar ausgespült wird und zur Konservierung Ethanol, Isopropanol oder Mischungen dieser Alkohole enthält.

Durch wiederholtes Blechen, Dauerwellen oder Färben, aber auch durch häufiges Waschen der Haare mit entfettenden Haarreinigungsmitteln, kommt es zu einer Schädigung der Haarstruktur. Das Haar wird spröde und verliert seinen Glanz. Weiterhin lädt sich das Haar beim Kämmen elektrostatisch auf und die aufgerauhte Haaroberfläche verursacht Verfilzungen und Verknotungen des Haares. Hierdurch wird das Kämmen sehr erschwert.

Haarbehandlungsmittel mit einer kämmbarkeitsverbessernden und pflegenden Wirkung, insbesondere in Form von Haarpflegeemulsionen, haben daher eine erhebliche Bedeutung erlangt.

Diese Haarpflegeemulsion liegen in der Regel als mit Wasser leicht ausspülbare Öl-in-Wasser-Emulsionen vor. Sie enthalten als Gerüstbestandteile halbfeste oder feste Substanzen, wie beispielsweise bestimmte Öle und Wachse. Als Emulgatoren werden hauptsächlich anionische oder kationische Tenside verwendet.

Derartige Haarpflegeemulsionen, welche auch als Cremerinses, Conditioner oder Haarkuren bezeichnet werden, besitzen im Gegenstanz zu Haarshampoos aufgrund ihres relativ geringen Tensidgehaltes keine reinigende oder entfettende Wirkung.

Während bei kationischen Haarpflegeemulsionen ein Zusatz von Konservierungsstoffen in der Regel nicht notwendig ist, da die verwendeten kationischen Tenside, besonders im sauren pH-Bereich, selbst konservierend wirken, müssen anionische Haarpflegeemulsionen durch den Zusatz von Konservierungsstoffen gegen den Befall durch Mikroorganismen geschützt werden. Als Konservierungsstoffe werden hierbei beispielsweise Formaldehyd, 5-Brom-5-nitro-2,3-dioxan, p-Hydroxybenzoesäureester, Salicylsäure, 2,4,4'-Trichlor-2'-hydroxy-diphenylether, 5-Chlor-2-methyl-3-isothiazolon, 2-Methyl-3-isothiazolon und 2-Brom-2-nitro-propandiol-(1,3) verwendet.

In letzter Zeit sind einige dieser Konservierungsstoffe, wie zum Beispiel Formaldehyd und 2-Methyl-3-isothiazolon, in den Verdacht geraten, in physiologischer und dermatologischer Hinsicht nicht völlig unbedenklich zu sein. Weiterhin ist von konservierend wirkenden Aldehyden und Phenolen bekannt, daß bei ihnen Reaktionen oder Wechselwirkungen mit Proteinen auftreten können.

Bei Aldehyden besteht zudem die Gefahr einer Sensibilisierung, während Phenole, wie zum Beispiel Salicylsäure, insbesondere in höheren Konzentrationen, keratolytisch wirken.

Aus den vorstehend genannten Gründen wird weltweit die Verwendung einer Vielzahl von Konservierungsstoffen immer stärker eingeschränkt oder gänzlich verboten.

Insbesondere bei anionischen Haarpflegeemulsionen, die zum einen in physiologischer und dermatologischer Hinsicht gut verträglich sein sollen und zum anderen ausreichend gegen mikrobiellen Befall geschützt sein müssen, ist eine befriedigende Konservierung daher oft äußerst problematisch.

Aufgabe der vorliegenden Erfindung ist es, eine stabile anionische Haarpflegeemulsion zur Verfügung zu stellen, welche einerseits hervorragende haarkonditionierende Eigenschaften besitzt und andererseits ausgezeichnet konserviert ist, ohne daß die Konservierung zu einer Beeinträchtigung der physiologischen Verträglichkeit der Haarpflegeemulsion führt.

Überraschenderweise wurde nunmehr gefunden, daß eine aniontensidhaltige Haapflegeemulsion, welche dadurch gekennzeichnet ist, daß siefrei von Konservierungsstoffen ist und 0,1 bis 6,5 Gewichtsprozent eines anionischen Tensides sowie 9 bis 18 Gewichtsprozent Ethanol, Isopropanol oder eines Ethanol/Isopropanol-Gemisches enthält, die gestellte Aufgabe in hervorragender Weise erfüllt.

Besonders überraschend ist hierbei, daß weder die Emulsionsbildung noch die Stabilität der Emulsion durch den Alkoholzusatz beeinträchtigt wird, obwohl Alkohole in der Literatur - zum Beispiel Houben-Weyl, ''Methoden der organischen Chemie, Bd. I/1 (1958), Seite 214 und 216; Deutsche Apotheker Zeitung 104 Jg., Nr. 39, Seite 1334, und Chinese Journal of Applied Chemistry, Vol. 3, No. 6 (1986), Seite 55 - 57 - als Emulsionsbrecher, das heißt als Hilfsmittel zur Trennung der Öligen und wäßrigen Phase von Emulsionen (Demulsifikation), empfohlen werden.

In der Literatur werden ferner zwar alkoholhaltige kosmetische Mittel beschrieben, jedoch enthalten alle diese Mittel außerdem Konservierungsmittel.

So ist aus dem Derwent-Abstract Hr. 85-26 16 73/42 der SU-A 1 148 616 ein Natriumlaurylethersulfat und Ethanol enthaltendes cremeförmiges Haarbehandlungsmittel bekannt, das jedoch durch den Zusatz von p-Hydroxybenzoesäuremethylester konserviert ist. Die EP-A 0 218 441 beschreibt kosmetische Zubereitungen, welche einen Pflanzenertrakt, einen Alkohol sowie eine bestimmte Konservierungsstoffkombination enthalten. Aus der Zusammenfassung Nr. 131 382 t, Chemical Abstracts 76, Seite 306 ist es bekannt, daß Kaliumsorbat - Alkohol Mischungen gegen einen bestimmten Bakterienstamm in einer Öl-in-Wasser Creme konservierend wirken. Ferner sind aus der EP-A 0 276 151 aniontensidhaltige Haarwuchsmittel auf der Basis des Wirkstoffs [Benzo-1,2,4-thiadiazin]-1,1-dioxid bekannt, die Isopropanol enthalten können. Sofern diese als Emulsion vorliegen, enthalten sie zusätzlich Konservierungsmittel.

Der Literatur konnte demzufolge keine Anregung entnommen werden, daß aniontensidhaltige Haarpflegeemulsionen durch den alleinigen Zusatz von Ethanol oder Isopropanol beziehungsweise deren Gemisch hervorragend konserviert werden, ohne daß hierdurch die Stabilität der Emulsion beeinträchtig wird.

Gegenstand der vorliegenden Erfindung ist eine aniontensidhaltige Haarpflegeemulsion, welche dadurch gekennzeichnet ist, daß sie frei von Konservierungsstoffen ist und 0,1 bis 6,5 Gewichtsprozent eines anionischen Tensides sowie 9 bis 18 Gewichtsprozent Ethanol, Isopropanol oder eines Ethanol/Isopropanol-Gemisches enthält.

Die erfindungsgemäße Haarpflegeemulsion enthält vorzugsweise 0,4 bis 4 Gewichtsprozent eines anionischen Tensides und 10 bis 14 Gewichtsprozent Ethanol, Isopropanol oder eines Ethanol/Isopropanol-Gemisches. Für die Verwendung in der erfindungsgemäßen Haarpflegeemulsion geeignete anionische Tenside sind zum Beispiel Alkali-, Erdalkali-, Ammonium-oder Alkaminsalze von Alkansulfonaten, Alkylbenzolsulfonaten, Alkylsulfaten, Alkylethersulfaten und Alkylethercarboxylaten, wie beispielsweise C₁₂- bis C₁₈-Alkylsulfatnatriumsalze, insbesondere Natriumcetylstearylsulfat und Natriumlaurylsulfat; anionische Fettsäurekondensationsprodukte, wie zum Beispiel das Kaliumsalz des Kondensationsproduktes aus Kokosfettsäurechlorid und tierischem Eiweißhydrolysat (Lamepon ® S der Firma Grünau); Seifen, wie beispielsweise Natrium-oder Kaliumstearat, sowie Dinatriumsalze der Sulfosuccinhalbester von Alkanolamiden. Vorzugsweise enthält die Haarpflegeemulsion neben dem anionischen Tensid keine weiteren Tenside. Sollten jedoch weitere Tenside enthalten sein, so sind diese, bezogen auf die Gewichtsmenge des anionischen Tensides, in einem deutlichen Unterschuß vorhanden.

Bei der erfindungsgemäßen Haarpflegeemulsion handelt es sich um eine Öl-in-Wasser-Emulsion, welche vorzugsweise 1 bis 16 Gewichtsprozent einer Ölkomponente sowie 59,5 bis 89,9 Gewichtsprozent Wasser enthält. Besonders bevorzugt sind solche Haarpflegeemulsionen, die 2 bis 10 Gewichtsprozent der Ölkomponente und 72 bis 87,5 Gewichtsprozent Wasser enthalten.

Als Ölkomponente können alle in Öl-in-Wasser-Emulsionen verwendbaren natürlichen oder synthetischen Öle und Wachse, alleine oder in Kombination, eingesetzt werden.

Geeignete Öle oder Wachse sind beispielsweise Paraffin-Öle und Isoparaffinöle; Siliconöle und Siliconwachse, insbesondere lineare Polydimethylsiloxane; Vaseline; Paraffinwachse und Ceresinwachse; höhere Fettalkohole, wie zum Beispiel Cetylalkohol und Stearylalkohol; Fettalkoholester, wie beispielsweise Cetyl- oder Stearylpalmitat; Fettsäureglyceride, wie zum Beispiel Glycerin-mono-di-stearat oder Glyceryltristearat; Lanolin; Lanolinöle; Lanolinalkohole sowie Bienenwachs.

Eine bevorzugte Ausführungsform der Erfindung ist eine Öl-in-Wasser-Haarpflegeemulsion, welche dadurch gekennzeichnet ist, daß sie frei von Konservierungsstoffen ist und
(a) 9 bis 18 Gewichtsprozent Ethanol, Isopropanol oder eines Ethanol/Isopropanol-Gemisches,
(b) 1 bis 16 Gewichtsprozent mindestens eines Öles oder Wachses,
(c) 0,1 bis 6,5 Gewichtsprozent eines anionischen Tensides und
(d) 59,5 bis 89,9 Gewichtsprozent Wasser
enthält.

Besonders bevorzugt ist eine Öl-in-Wasser-Haarpflegeemulsion, welche dadurch gekennzeichnet ist, daß sie frei von Konservierungsstoffen ist und
(a) 10 bis 14 Gewichtsprozent Ethanol, Isopropanol oder eines Ethanol/Isopropanol-Gemisches,
(b) 2 bis 10 Gewichtsprozent mindestens eines Öles oder Wachses,
(c) 0,4 bis 4 Gewichtsprozent eines anionischen Tensides und
(d) 72 bis 87.5 Gewichtsprozent Wasser
enthält.

Das Gewichtsverhältnis von Ölkomponente zu Wasser beträgt in der erfindungsgemäßen Haarpflegeemulsion vorzugsweise 1 : 4 bis 1 : 100 , wobei ein Gewichtsverhältnis von 1 : 8 bis 1 : 43 besonders bevorzugt ist. Das anionische Tensid und die Ölkomponente sollen in der erfindungsgemäßen Haarpflegeemulsion vorzugsweise in einem Gewichtsverhältnis von 1 : 1 bis 1 : 13 vorliegen, wobei ein Gewichtsverhältnis von 1 : 1 bis 1 : 11,5 besonders bevorzugt ist.

Die erfindungsgemäße Haarpflegeemulsion kann weiterhin alle für ein derartiges Mittel üblichen und bekannten Zusätze, wie beispielsweise Parfümöle, Verdicker, Antioxidantien, Wirkstoffe gegen Kopfschuppen, Kräuterektrakte, haarpflegende Substanzen, zum Beispiel Cholesterin und Betain, sowie kosmetische Farbstoffe enthalten und sowohl in flüssiger als auch pastöser Form vorliegen.

Die aniontensidhaltige Haarpflegeemulsion gemäß der vorliegenden Erfindung ist nicht sensibilisierend und besitzt eine gute Hautverträglichkeit. Durch den Alkoholgehalt wird, eine gute Konservierung der Haarpflegeemulsion erreicht, ohne die Stabilität oder physiologische Verträglichkeit der Haarpflegeemulsion zu beeinträchtigen. Weiterhin werden durch den Alkoholzusatz die kämmbarkeitsverbessernden Eigenschaften der Haarpflegeemulsion, insbesondere bei feuchtem Haar, deutlich verbessert.

Ein weiterer Gegenstand der vorliegenden Erfindung ist daher die Verwendung von 9 bis 18 Gewichtsprozent Ethanol, Isopropanol oder eines Ethanol/Isopropanol-Gemisches zur alleinigen Konservierung von aniontensidhaltigen Haarpflegeemulsionen.

Die Anwendung der erfindungsgemäßen Haarpflegeemulsion erfolgt in üblicher Weise, indem im Anschluß an eine Haarwäsche eine ausreichende Menge der Haarpflegeemulsion, je nach Haarlänge etwa 5 bis 20 Gramm, im handtuchtrockenen Haar verteilt wird und nach einer Einwirkungszeit von etwa 2 bis 15 Minuten mit lauwarmem Wasser wieder ausgespült wird.

Die nachfolgenden Beispiele sollen den Gegenstand der Erfindung näher erläutern, ohne den Gegenstand auf diese Beispiele zu beschränken.

### Beispiele

| **Beispiel 1: Cremerinse** | |
|---|---|
| 12,0 g | Ethanol |
| 2,0 g | Cetylstearylalkohol |
| 0,4 g | Natriumlaurylsulfat |
| 85,6 g | Wasser |
| 1̅0̅0̅,̅0̅ g̅ | |

| **Beispiel 2: Haarkur (pastös)** | |
|---|---|
| 12,00 g | Ethanol |
| 4,96 g | Cetylstearylalkohol |
| 3,10 g | Lanolin |
| 1,71 g | Glycerin-mono-di-stearat |
| 1,14 g | Natriumcetylstearylsulfat |
| 0,09 g | Kaliumstearat |
| 0,15 g | Cholesterin |
| 76,85 g | Wasser |
| 1̅0̅0̅,̅0̅0̅ g̅ | |

| **Beispiel 3: Cremerinse** | |
|---|---|
| 5,00 g | Ethanol |
| 5,00 g | Isopropanol |
| 2,48 g | Cetylstearylalkohol |
| 0,86 g | Glycerin-mono-di-stearat |
| 0,60 g | Lanolin |
| 3,72 g | Natriumcetylstearylsulfat |
| 0,05 g | Kaliumstearat |
| 82,29 g | Wasser |
| 1̅0̅0̅,̅0̅0̅ g̅ | |

| **Beispiel 4: Haarkur (pastös)** | |
|---|---|
| 10,0 g | Isopropanol |
| 2,6 g | Cetylstearylalkohol |
| 1,5 g | Lanolin |
| 0,5 g | Natriumcetylstearylsulfat |
| 10,0 g | Betainmonohydrat |
| 75,4 g | Wasser |
| 1̅0̅0̅,̅0̅ g̅ | |

| **Beispiel 5: Haartönende Haarpflegeemulsion** | |
|---|---|
| 13,00 g | Ethanol |
| 2,40 g | Cetylstearylalkohol |
| 2,20 g | Vaseline |
| 0,40 g | Natriumcetylstearylsulfat |
| 10,00 g | Betainmonohydrat |
| 0,10 g | Disperse Blue 3 (C.I. 61 505) |
| 0,05 g | 2-Amino-4-nitro-phenol |
| 71,85 g | Wasser |
| 1̅0̅0̅,̅0̅0̅ g̅ | |

## Patentansprüche

1. Aniontensidhaltige Haarpflegeemulsion, dadurch gekennzeichnet, daß sie frei von Konservierungsstoffen ist und 0,1 bis 6,5 Gewichtsprozent eines anionischen Tensides sowie 9 bis 18 Gewichtsprozent Ethanol, Isopropanol oder eines Ethanol/Isopropanol-Gemisches enthält.

2. Haarpflegeemulsion nach Anspruch 1, dadurch gekennzeichnet, daß das anionische Tensid ausgewählt ist aus Alkali-, Erdalkali-, Ammonium- oder Alkaminsalzen von Alkansulfonaten, Alkylbenzolsulfonaten, Alkylsulfaten, Alkylethersulfaten und Alkylethercarboxylaten; anionischen Fettsäurekondensationsprodukten; Seilen und Dinatriumsalzen der Sulfosuccinhalbester von Alkanolamiden.

3. Haarpflegeemulsion nach Anspruch 1 oder 2, dadurch gekennzeichnet, daß sie in Form einer Öl-in-Wasser-Emulsion vorliegt und 1 bis 16 Gewichtsprozent einer Ölkomponente enthält.

4. Haarpflegeemulsion nach Anspruch 3, dadurch gekennzeichnet, daß die Ölkomponente ausgewählt ist aus Paraffin- und Isoparaffinölen; Siliconölen und Siliconwachsen; Vaseline; Paraffin- und Ceresinwachsen; höheren Fettalkoholen; Fettalkoholestern; Fettsäureglyceriden; Lanolin; Lanolinölen; Lanolinalkoholen sowie Bienenwachs.

5. Öl-in-Wasser-Haarpflegeemulsion, dadurch gekennzeichnet, daß sie frei von Konservierungsstoffen ist und
(a) 9 bis 18 Gewichtsprozent Ethanol, Isopropanol oder eines Ethanol/Isopropanol-Gemisches,
(b) 1 bis 16 Gewichtsprozent mindestens eines Öles oder Wachses,
(c) 0,1 bis 6,5 Gewichtsprozent eines anionischen Tensides und
(d) 59,5 bis 89,9 Gewichtsprozent Wasser
enthält.

6. Öl-in-Wasser-Haarpflegeemulsion, dadurch gekennzeichnet, daß sie frei von Konservierungsstoffen ist und
(a) 10 bis 14 Gewichtsprozent Ethanol, Isopropanol oder eines Ethanol/Isopropanol-Gemisches,
(b) 2 bis 10 Gewichtsprozent mindestens eines Öles oder Wachses,
(c) 0,4 bis 4 Gewichtsprozent eines anionischen Tensides und
(d) 72 bis 87,5 Gewichtsprozent Wasser
enthält

7. Haarpflegeemulsion nach einem der Ansprüche 1 bis 6, dadurch gekennzeichnet, daß das Gewichtsverhältnis von Ölkomponente zu Wasser 1 : 4 bis 1 : 100 beträgt.

8. Haarpflegeemulsion nach einem der Ansprüche 1 bis 7, dadurch gekennzeichnet, daß das Gewichtsverhältnis von anionischem Tensid zu Ölkomponente 1 : 1 bis 1 : 13 beträgt.

9. Verwendung von 9 bis 18 Gewichtsprozent Ethanol, Isopropanol oder eines Ethanol/Isopropanol-Gemisches zur alleinigen Konservierung von aniontensidhaltigen Haarpflegeemulsionen.

## Claims

1. Anionic surfactant - containing hair care emulsion, characterised in that it is free of preservation agents and contains 0.1 - 6.5 weight percent of an anionic surfactant as well as 9 - 18 weight percent ethanol, isopropanol or an ethanol-isopropanol mixture.

2. Hair care emulsion according to claim 1, characterised in that the anionic surfactant is selected from alkali, alkaline earth, ammonium or alkamine salts of alkane sulphonates, alkyl benzene sulphonates, alkyl sulphates, alkyl ether sulphates and alkyl ether carboxylates; anionic fatty acid condensation products; soaps and disodium salts of sulphosuccinic acid half esters of alkanolamides.

3. Hair care emulsion according to claim 1 or 2, characterised in that it is in the form of an oil-in-water emulsion and contains 1 - 16 weight percent of an oil component.

4. Hair care emulsion according to claim 3, characterised in that the oil component is selected from paraffin and isoparaffin oils; silicone oils and silicone waxes; vaseline; paraffin and ceresin waxes; higher fatty alcohols; fatty alcohol esters; fatty acid glycerides; lanoline; lanoline oils; lanoline alcohols as well as beeswax.

5. Oil-in-water hair care emulsion, characterised in that it is free of preservation agents and contains:
(a) 9 - 18 weight percent ethanol, isopropanol or an ethanol/isopropanol mixture;
(b) 1 - 16 weight percent of at least one oil or wax;
(c) 0.1 - 6.5 weight percent of an anionic surfactant;
and
(d) 59.5 - 89.9 weight percent water.

6. Oil-in-water hair care emulsion, characterised in that it is free of preservation agents and contains:
(a) 10 - 14 weight percent ethanol, isopropanol or an ethanol/isopropanol mixture;
(b) 2 - 10 weight percent of at least one oil or wax;
(c) 0.4 - 4 weight percent of an anionic surfactant;
and
(d) 72 - 87.5 weight percent water.

7. Hair care emulsion according to any one of claims 1 to 6, characterised in that the weight ratio of the oil component to water is 1:4 to 1:100.

8. Hair care emulsion according to any one of claims 1 to 7, characterised in that the weight ratio of the anionic surfactant to oil component is 1:1 to 1:13.

9. Use of 9 - 18 weight percent ethanol, isopropanol or an ethanol/isopropanol mixture for the preservation of anionic surfactant - containing hair care emulsions only.

## Revendications

1. Emulsion de soins capillaires contenant des agents tensioactifs anioniques, caractérisée en ce qu'elle ne contient pas d'agents conservateurs et en ce qu'elle contient de 0,1 à 6,5 % en masse d'un agent tensioactif anionique ainsi que de 9 à 18 % en masse d'éthanol, d'isopropanol, ou d'un mélange éthanol-isopropanol.

2. Emulsion de soins capillaires selon la revendication 1, caractérisée en ce que l'agent tensioactif anionique est choisi parmi les sels alcalins, alcalino-terreux, d'ammonium et d'alkylamines de sulfonates d'alkyle,de sulfonates d'alkylbenzène, de sulfates d'alkyle, de sulfates d'alkyléther, et de carboxylates d'alkyléther, les produits de condensation d'acides gras; savons et sels disodiques de l'hémiester sulfosuccinique d'alcanolamides.

3. Emulsion de soins capillaires selon la revendication 1 ou 2, caractérisée en ce qu'elle se présente sous forme d'une émulsion huile dans l'eau et contient de 1 à 16 % en masse d'un dérivé d'huile.

4. Emulsion de soins capillaires selon la revendication 3, caractérisée en ce que le dérivé d'huile est choisi parmi les huiles de paraffine et d'isoparaffines, les huiles de silicones, et les cires de silicones, la vaseline, les cires de paraffine et de cérésine, les alcools gras de rang élevé, les esters d'alcools gras, les glycérides d'acides gras, la lanoline, les huiles de lanoline, les alcools de lanoline, ainsi que la cire d'abeille.

5. Emulsion de soins capillaires du type huile dans l'eau, caractérisée en ce qu'elle ne contient pas d'agents conservateurs et en ce qu'elle contient:
(a) de 9 à 18 % en masse d'éthanol, d'isopropanol ou d'un mélange ethanol-isopropanol;
(b) de 1 à 16 % en masse d'au moins une huile ou une cire;
(c) de 0,1 à 6,5 % en masse d'un agent tensioactif anionique;
(d) de 59,5 à 89,9 % en masse d'eau.

6. Emulsion de soins capillaires du type huile dans l'eau, caractérisée en ce qu'elle ne contient pas d'agents conservateurs et en ce qu'elle contient:
(a) de 10 à 14 % en masse d'éthanol, d'isopropanol ou d'un mélange éthanol-isopropanol;
(b) de 2 à 10 % en masse d'au moins une huile ou une cire;
(c) de 0,4 à 4 % en masse d'un agent tensioactif anionique;
(d) de 72 à 87,5 % en masse d'eau.

7. Emulsion de soins capillaires selon l'une des revendications 1 à 6, caractérisée en ce que la proportion pondérale de l'agent tensioactif anionique par rapport à l'eau va de 1:4 à 1:100.

8. Emulsion de soins capillaires selon l'une des revendications 1 à 7, caractérisée en ce que la proportion pondérale de l'agent tensioactif anionique par rapport aux composants huileux va de 1:1 à 1:13.

9. Utilisation de 9 à 18 % en masse d'éthanol, isopropanol ou un mélange éthanol-isopropanol pour la seule conservation d'émulsions de soins capillaires contenant des agents tensioactifs.
